# DEMANDE DE BREVET EUROPEEN

(11) **EP 4 344 750 A1**
(43) Date de publication de la demande: **03.04.2024**
(21) Numéro de dépôt: 23197484.1
(22) Date de dépôt: 14.09.2023
(51) Int. Cl.: A61Q 17/04, A61K 8/31, A61K 8/36, A61K 8/41, A61K 8/49, A61K 8/67, A61K 8/92

(54) **COMPOSITION COSMÉTIQUE OU DERMATOLOGIQUE UTILE POUR LA PROTECTION SOLAIRE OU LE SOIN DE LA PEAU**

(30) Priorité: 15.09.2022 FR 2209310
(71) Demandeur: Rivet, Jean-Paul, 74290 Menthon Saint Bernard (FR)
(72) Inventeur: Rivet, Jean-Paul, 74290 Menthon Saint Bernard (FR)
(74) Mandataire: Lavoix

(57) **Abrégé**

La demande concerne une composition cosmétique ou dermatologique utile pour la protection solaire comprenant :
- à titre de filtres solaires : de l'ethylhexyl triazone, du diethylamino hydroxybenzoyl hexyl benzoate, et du bis-ethylhexyloxyphenol methoxyphenyl triazine,
- à titre de vitamines ou précurseur de vitamines : du tocopherol, un ester de celui-ci ou un mélange de ceux-ci, de la vitamine F, un éther, ester et/ou ester phosphorique de vitamine C, un ester de vitamine E, et du beta-carotene, et
- à titre d'huiles : de l'huile d'olive, de l'huile de coco et de l'huile de bourrache.

## Description

La présente invention concerne une composition cosmétique ou dermatologique utile pour la protection solaire et/ou le soin de la peau.

De nombreuses compositions solaires ou pour le soin de la peau sont disponibles sur le marché.

Ces compositions comprennent au moins un filtre solaire, également appelé filtre ultraviolet. De nombreuses compositions commerciales contiennent du dioxyde de titane à titre de filtre solaire. Le dioxyde de titane est suspecté d'avoir un impact négatif sur la santé, voire même d'être cancérigène lorsqu'il est sous forme nanométrique. On cherche à développer des compositions comprenant des filtres solaires alternatifs au dioxyde de titane. Les filtres solaires acceptables en cosmétique sont listés à l'annexe VI du règlement CE 1223/2009.

En outre, l'impact néfaste du soleil dépend de l'hydratation de la peau. La peau réagit d'autant mieux à une exposition au soleil qu'elle est hydratée. On recherche donc une composition qui permette non seulement de protéger la peau des rayons ultraviolets, mais aussi d'hydrater la peau.

A cet effet, selon un premier objet, la demande concerne une composition cosmétique ou dermatologique comprenant :
- à titre de filtres solaires : de l'ethylhexyl triazone, du diethylamino hydroxybenzoyl hexyl benzoate et du bis-ethylhexyloxyphenol methoxyphenyl triazine,
- à titre de vitamines ou précurseur de vitamines :
   du tocopherol, un ester de celui-ci ou un mélange de ceux-ci,
   de la vitamine F,
   un éther de vitamine C, un ester de vitamine C, un ester phosphorique de vitamine C ou un mélange de ceux-ci, et
   du beta-carotene, et
- à titre d'huiles : de l'huile d'olive, de l'huile de coco et de l'huile de bourrache.

La composition selon l'invention comprend un mélange de filtres solaires, d'huiles et de vitamines et de précurseur de vitamines. Cette composition permet avantageusement une bonne protection solaire et une excellente hydratation de la peau et, ce qui rend la peau plus apte au bronzage, et lui confère, lorsqu'elle est exposée au soleil, une couleur de bronzage particulière et recherchée.

La composition peut comprendre d'autre(s) filtre(s) solaire(s), vitamine(s) ou précurseur(s) et huile(s) que ceux mentionnés ci-dessus.

Sauf mention contraire, les proportions massiques en composants sont par rapport au poids total de la composition. Les ratios massiques sont le ratio de la masse d'un premier composant par rapport à la masse du second composant.

Les modes de réalisation décrits ci-dessous peuvent être combinés entre eux.

La composition comprend de l'ethylhexyl triazone, du diethylamino hydroxybenzoyl hexyl benzoate, et du bis-ethylhexyloxyphenol methoxyphenyl triazine à titre de filtres solaires.

Ces filtres solaires sont de préférence présents :
- dans une proportion de de 0,5% à 8,0% en poids, de préférence de 1,0 à 6,0% en poids, d'ethylhexyl triazone,
- dans une proportion de diethylamino hydroxybenzoyl hexyl benzoate telle que le ratio massique du diethylamino hydroxybenzoyl hexyl benzoate sur l'ethylhexyl triazone est de 0,3 à 1,5, de préférence de 0,4 à 1,3, et
- dans une proportion de bis-ethylhexyloxyphenol methoxyphenyl triazine telle que le ratio massique du bis-ethylhexyloxyphenol methoxyphenyl triazine sur l'ethylhexyl triazone est de 0,1 à 1,5, de préférence de 0,2 à 1,1.

Ces filtres solaires, en particulier dans les proportions ci-dessus, permettent de conférer une bonne protection solaire. Plus la concentration en filtres solaires est élevée, plus le coefficient de protection solaire (« sun protection factor » (SPF) en anglais) est élevé.

La composition peut comprendre d'autres filtres solaires, par exemple de l'ethylhexylsalicylate et/ou de l'ethylhexyl dimethyl PABA.

La proportion d'ethylhexylsalicylate est de préférence telle que le ratio massique de l'ethylhexylsalicylate sur l'ethylhexyl triazone est de 0,2 à 2,0, de préférence de 0,5 à 1,5.

La proportion d'ethylhexyl dimethyl PABA est de préférence telle que le ratio massique de l'ethylhexyl dimethyl PABA sur l'ethylhexyl triazone est de 0,1 à 1,5, de préférence de 0,3 à 1,3.

Généralement, au sein de la composition, la proportion cumulée en filtres solaires est de 1,0 à 30,0%, de préférence de 2,0 à 25,0% en poids, en particulier de 3,0 à 25,0%.

De préférence, la composition est exempte d'oxyde de zinc et/ou de dioxyde de titane.

De préférence, la composition est exempte de nanoparticules de taille moyenne de 1 à 100 nm, telle que mesurée par microscopie au balayage électronique, et en particulier exempte de nanoparticules de d'oxyde de zinc et/ou de dioxyde de titane ayant une telle taille. De préférence, la composition est exempte de nanomatériau, à savoir de matériau insoluble ou bio-persistant, fabriqué intentionnellement et se caractérisant par une ou plusieurs dimensions externes, ou une structure interne, sur une échelle de 1 à 100 nm tel que défini dans le règlement CE 1223/2009.

Généralement, la composition ne comprend pas d'octocrylene. De préférence, la composition ne comprend pas d'autres filtres solaires que l'ethylhexyl triazone, le diethylamino hydroxybenzoyl hexyl benzoate, le bis-ethylhexyloxyphenol methoxyphenyl triazine, l'ethylhexylsalicylate et le l'ethylhexyl dimethyl PABA, voire même pas d'autres filtres solaires que l'ethylhexyl triazone, le diethylamino hydroxybenzoyl hexyl benzoate et le bis-ethylhexyloxyphenol methoxyphenyl triazine.

La composition comprend des vitamines (le tocopherol est de la vitamine E ; la vitamine F) et des précurseurs ou dérivés de vitamines (l'ester de tocophérol est un précurseur de vitamine E ; le beta-carotene est un précurseur de la vitamine A ; l'éther, l'ester ou l'ester phosphorique de vitamine C sont des dérivés de la vitamine C).

Le tocophérol peut être présent sous forme d'un ester, de préférence choisi parmi le tocopherol acetate, le tocopherol linoleate ou d'un mélange de ceux-ci. La composition comprend de préférence un mélange de tocophérol et d'ester(s) de tocophérol.

La vitamine C est peu stable à la lumière, à la chaleur et au contact de l'eau. On préfère donc éviter de l'utiliser et la remplacer par des dérivés plus stables. Ainsi, la vitamine C est présente sous forme d'éther de vitamine C, d'ester de vitamine C, d'ester phosphorique de vitamine C ou de mélange de ceux-ci. L'ascorbyl glucoside et le 3-o-ethyl-L-ascorbic acid sont des exemples d'éther de vitamine C. Le magnésium ascorbyl phosphate ou le sodium ascorbyl phosphate sont des exemples d'ester phosphorique de vitamine C. L'ascorbyl palmitate, l'ascorbyl tetra isopalmitate sont des exemples d'ester de vitamine C. De préférence, le dérivé de vitamine C de la composition est du sodium ascorbyl phosphate.

La vitamine F correspond à un mélange d'acide linoléique et d'acide α-linolénique. Certaines huiles peuvent être utilisées comme source de vitamine F, par exemple l'huile de maïs (Zea Mays (Corn) oil), l'huile de rose musquée (Rosa Moschata Seed oil) ou un mélange de celles-ci.

Ces vitamines et précurseurs sont de préférence présents dans une proportion de :
- de 0,9 à 2,0% en poids, de préférence de 1,0 à 1,6% en poids, de tocopherol, et/ou
- de 0,1 à 2,0% en poids, de préférence de 0,2 à 1,5% en poids, d'ester de tocopherol, et/ou

- de 0,2 à 1,5 % en poids, de préférence de 0,5 à 1,3% en poids, de vitamine F, et/ou
- de 0,1 à 1,0% en poids, de préférence de 0,2 à 0,4% en poids, d'éther de vitamine C, d'ester de vitamine C, d'ester phosphorique de vitamine C ou de mélange de ceux-ci, et/ou
- de 0,0001 à 0,01 en poids, de préférence de 0,0005 à 0,005% en poids, de beta-carotene.

En plus des vitamines ou précurseurs mentionnés ci-dessus, la composition peut comprendre du panthenol, de préférence de 0,1 à 2,0% en poids, de préférence de 0,4 à 1,0% en poids, de panthenol.

Généralement, au sein de la composition, la proportion cumulée en vitamines et précurseurs est de 2,0 à 5,0% en poids, de préférence de 2,5 à 4,5% en poids.

De préférence, la composition est exempte de dérivés de vitamine A choisis parmi l'acide rétinoïque, le rétinol, le rétinal et le rétinaldéhyde. Ces dérivés peuvent en effet être irritants pour la peau.

La composition comprend également de l'huile d'olive, de l'huile de coco et de l'huile de bourrache. L'huile d'olive nourrit et hydrate. En particulier, l'huile d'olive combat la perte en eau de la peau, et restaure la barrière cutanée. L'huile de coco est très nourrissante, aide à lutter contre la déshydratation, apaise, calme les rougeurs, soulage les peaux échauffées par le soleil. L'huile de bourrache a des propriétés hydratantes, régénérantes et anti-âge.

Ces huiles sont de préférence présentes au sein de la composition dans une proportion de :
- 0,5 à 5,0% en poids, de préférence de 1,0 à 4,0% en poids, d'huile d'olive, et/ou
- 0,5 à 10,0% en poids, de préférence de 1,0 à 7,0% en poids, d'huile de coco, et/ou
- 0,1 à 2,0% en poids, de préférence de 0,2 à 1,0% en poids, d'huile de bourrache.

La composition peut également comprendre, en plus des huiles ci-dessus, de l'huile de sésame et/ou de l'huile d'onagre. Ces huiles sont de préférence présentes dans une proportion de :
- de 0,5 à 5% en poids, de préférence de 1,0 à 3,0% en poids d'huile de sésame, et/ou
- de 0,1 à 2,0% en poids, de préférence de 0,2 à 1,0% en poids, d'huile d'onagre.

Généralement, au sein de la composition, la proportion cumulée en huile d'olive, huile de coco, huile de bourrache, huile de sésame et huile d'onagre est de 3,0 à 20,0%, de préférence de 4,0 à 15,0% en poids.

Au sens de la demande, lorsqu'une concentration en vitamine F est précisée, c'est sans tenir compte des proportions en acide linoléique et en acide α-linolénique contenus dans les huiles d'olive, de coco, de bourrache, de sésame et d'onagre. Généralement, la composition comprend une autre huile comme source de vitamine F, par exemple l'huile de maïs (Zea Mays (Corn) oil), l'huile de rose musquée (Rosa Moschata Seed oil) ou un mélange de celles-ci. Les proportions en vitamine F mentionnées ci-dessus (de 0,2 à 1,5 % en poids, de préférence de 0,5 à 1,3% en poids) correspondent aux proportions en acide linoléique et en acide α-linolénique apportée par cette(ces) autre(s) huile(s).

Les huiles utilisées dans la composition sont de préférence d'origine végétale. De préférence, la composition est exempte d'huile paraffinum liquidum, parraffinum cera et/ou microcristallina, voire même plus généralement exempte d'huile minérale.

La composition peut en outre comprendre de l'aloe vera ou un extrait de celui-ci, par exemple un jus déshydraté d'aloe vera.

La composition peut comprendre des additifs supplémentaires choisis parmi un agent de contrôle de la viscosité, un agent conservateur, un émollient, un agent d'hydratation, un tensioactif, un parfum, un acide ou une base pour ajuster le pH, un conditionneur de peau, un agent masquant et des mélanges de ceux-ci.

De préférence, la composition est exempte d'acide ethylenediaminetetraacetique (EDTA), sous forme acide ou de sel, dont l'impact environnemental est très controversé. De préférence, la composition est exempte d'agent complexant les métaux lourds.

De préférence, la composition est exempte d'hydroxytoluène butylé (BHT), ce dernier étant suspecté d'être un perturbateur endocrinien voire probablement cancérigène.

De préférence, la composition est exempte de polysiloxanes (également appelée silicones), et en particulier exempte de dimethicone (polydimethylsiloxane), de cyclopentasiloxane et de decamethylcyclopentasiloxane (également appelé cyclomethicone), dont l'impact environnemental est très controversé, en particulier car ils sont peu biodégradables.

De préférence, la composition est exempte de propylène glycol, afin de limiter les composants issus de la pétrochimie.

La composition comprend généralement un solvant. La composition se présente sous forme d'un gel aqueux ou huileux, de préférence huileux, d'une émulsion de type E/H ou H/E ou d'un liquide monophasique.

Selon une première alternative, la composition est sous forme d'une émulsion huile dans l'eau.

Les modes de réalisation décrits ci-dessous pour la première alternative peuvent être combinés entre eux.

Selon cette première alternative, de préférence, les seuls filtres solaires de la composition sont l'ethylhexyl triazone, le diethylamino hydroxybenzoyl hexyl benzoate, et le bis-ethylhexyloxyphenol methoxyphenyl triazine.

Selon cette première alternative, de préférence, les filtres solaires sont tels que :
- la proportion de 0,5% à 6,0% en poids, de préférence de 1,0 à 5,0% en poids, d'ethylhexyl triazone,
- la proportion de diethylamino hydroxybenzoyl hexyl benzoate telle que le ratio massique du diethylamino hydroxybenzoyl hexyl benzoate sur l'ethylhexyl triazone est de 0,7 à 1,4, de préférence de 0,9 à 1,3, et
- la proportion de bis-ethylhexyloxyphenol methoxyphenyl triazine, telle que le ratio massique du bis-ethylhexyloxyphenol methoxyphenyl triazine sur l'ethylhexyl triazone est de 0,1 à 1,5, de préférence de 0,2 à 1,1.

Selon cette première alternative, de préférence, la composition comprend du panthenol.

Selon cette première alternative, de préférence, la composition est exempte d'huile de sésame et/ou de d'huile d'onagre.

Selon cette première alternative, de préférence, les huiles sont de préférence présentes dans une proportion de :
- 0,5 à 4,0% en poids, de préférence de 1,0 à 3,0% en poids, d'huile d'olive, et/ou
- 0,5 à 4,0% en poids, de préférence de 1,0 à 3,0% en poids, d'huile de coco, et/ou
- 0,1 à 2,0% en poids, de préférence de 0,2 à 1,0% en poids, d'huile de bourrache.

Généralement, au sein de la composition selon cette première alternative, la proportion cumulée en huile d'olive, huile de coco et huile de bourrache est de 3,0 à 6,0%, de préférence de 4,0 à 5,0% en poids.

La composition selon cette première alternative comprend de préférence de l'aloe vera ou un extrait de celui-ci, par exemple un jus déshydraté d'aloe vera.

Selon cette première alternative, de préférence, la proportion massique en eau est de 40 à 70%, de préférence de 50 à 60% en poids.

Selon une seconde alternative, la composition est sous forme d'une huile monophasique.

Les modes de réalisation décrits ci-dessous pour la seconde alternative peuvent être combinés entre eux.

Selon cette seconde alternative, de préférence, les seuls filtres solaires de la composition sont l'ethylhexyl triazone, le diethylamino hydroxybenzoyl hexyl benzoate, et le bis-ethylhexyloxyphenol methoxyphenyl triazine, ou bien les seuls filtres solaires de la composition sont l'ethylhexyl triazone, le diethylamino hydroxybenzoyl hexyl benzoate, le bis-ethylhexyloxyphenol methoxyphenyl triazine l'ethylhexylsalicylate et l'ethylhexyl dimethyl PABA.

Selon cette seconde alternative, de préférence, les filtres solaires sont de préférence tels que :
- la proportion de 1,0% à 6,0% en poids, de préférence de 1,0 à 5,5% en poids, d'ethylhexyl triazone,
- la proportion de diethylamino hydroxybenzoyl hexyl benzoate est telle que le ratio massique du diethylamino hydroxybenzoyl hexyl benzoate sur l'ethylhexyl triazone est de 0,2 à 1,0, de préférence de 0,3 à 0,9, et
- la proportion de bis-ethylhexyloxyphenol methoxyphenyl triazine est telle que le ratio massique du bis-ethylhexyloxyphenol methoxyphenyl triazine sur l'ethylhexyl triazone est de 0,1 à 1,2, de préférence de 0,2 à 1,1,
- la proportion l'ethylhexylsalicylate est telle que le ratio massique de l'ethylhexylsalicylate sur l'ethylhexyl triazone est de 0 (ethylhexylsalicylate absent) ou de 0,7 à 1,3,
- la proportion d'ethylhexyl dimethyl PABA telle que le ratio massique de l'ethylhexyl dimethyl PABA sur l'ethylhexyl triazone est de 0 (ethylhexyl dimethyl PABA absent) ou de de 0,5 à 1,1.

Selon cette seconde alternative, de préférence, la composition est exempte de panthenol.

Selon cette seconde alternative, de préférence, la composition comprend de l'huile de sésame et de d'huile d'onagre.

Selon cette seconde alternative, de préférence, les huiles sont de préférence présentes dans une proportion :
- de 1,0 à 5,0% en poids, de préférence de 2,0 à 4,0% en poids, d'huile d'olive, et/ou
- de 4,0 à 8,0% en poids, de préférence de 5,0 à 7,0% en poids, d'huile de coco, et/ou
- de 0,1 à 2,0% en poids, de préférence de 0,2 à 1,0% en poids, d'huile de bourrache, et/ou
- de 0,5 à 5% en poids, de préférence de 1,0 à 3,0% en poids d'huile de sésame, et/ou
- de 0,1 à 2,0% en poids, de préférence de 0,2 à 1,0% en poids, d'huile d'onagre.

Généralement, au sein de la composition selon cette seconde alternative, la proportion cumulée en huile d'olive, huile de coco, huile de bourrache, huile de sésame et huile d'onagre est de 9,0 à 15,0%, de préférence de 11,0 à 13,0% en poids.

La composition peut être préparée par simple mélange de ses constituants.

Selon un deuxième objet, l'invention concerne l'utilisation de la composition pour la protection solaire et/ou le soin de la peau. La composition selon l'invention peut être appliquée sur le visage et/ou sur le corps.

L'invention est illustrée au vu des exemples qui suivent.

### Exemples

Des crèmes de soin solaires ont été préparées ayant la composition fournie au tableau 1.

Des huiles solaires ont été préparées ayant la composition fournie au tableau 2.

Il a été observé que ces crèmes et huiles permettent un excellente hydratation de la peau et une bonne protection solaire.

**[Tableau 1]**

| Exemple | | A | B | C | D | E | F |
|---|---|---|---|---|---|---|---|
| Protection solaire | | Faible | Faible | moyenne | Haute | Haute | Très haute |
| filtres UV | ethylhexyl triazone | 1% | 1% | 2% | 3% | 4,50% | 4,50% |
| | diethylamino hydroxybenzoyl hexyl benzoate | 1% | 1% | 2% | 3% | 4,50% | 5% |
| | bis-ethylhexyloxyphenol methoxyphenyl triazine | 0,25% | 1% | 2% | 3% | 3% | 3% |
| vitamines | tocopherol (vitamine E) | 1,51% | 1,10% | 1,10% | 1,10% | 1,10% | 1,10% |
| | panthenol ( vitamine B5) | 0,60% | 0,80% | 0,80% | 0,80% | 0,80% | 0,80% |
| | vitamine F | 0,60% | 0,60% | 0,60% | 0,60% | 0,60% | 0,60% |
| | sodium ascorbyl phosphate | 0,30% | 0,30% | 0,30% | 0,30% | 0,30% | 0,30% |
| | tocopherol acetate et/ou tocopherol linoleate | 0,40% | 1,22% | 1,22% | 1,22% | 1,22% | 1,22% |
| | beta carotene (provitamine A) | 0,003% | 0,001% | 0,001 % | 0,001% | 0,001% | 0,001% |
| huiles | huile olive | 2% | 2% | 2% | 2% | 2% | 2% |
| | huile coco | 2% | 2% | 2% | 2% | 2% | 2% |
| | huile bourrache | 0,50% | 0,50% | 0,50% | 0,50% | 0,50% | 0,50% |
| actif | aloe vera (à base de jus déshydraté) | 2% | 2% | 2% | 2% | 2% | 2% |
| parfum | | ND | ND | ND | ND | ND | ND |

| | | | | | | | |
|---|---|---|---|---|---|---|---|
| Compositions des crèmes de soin solaires, les proportions étant en poids par rapport au poids total de la crème | | | | | | | |

**[Tableau 2]**

| Exemples | | G | H | I | J | K |
|---|---|---|---|---|---|---|
| Protection solaire | | Faible | Faible | moyenne | Haute | Haute |
| filtres UV | ethylhexyl triazone | 1,50% | 2% | 3% | 5% | 5% |
| | diethylamino hydroxybenzoyl hexyl benzoate | 1% | 1% | 2% | 3% | 3% |
| | bis-ethylhexyloxyphenol methoxyphenyl triazine | 0,50% | 2% | 2% | 4% | 5% |
| | ethylhexyl salicylate | - | - | - | - | 5% |
| | ethylhexyl dimethyl PABA | - | - | - | - | 4% |
| vitamines | tocopherol (vitamine E) | 1,40% | 1,40% | 1,40% | 1,40% | 1,40% |
| | vitamine F | 1,20% | 1,20% | 1,20% | 1,20% | 1,20% |
| | sodium ascorbyl phosphate | 0,30% | 0,30% | 0,30% | 0,30% | 0,30% |
| | tocopherol acetate | 0,40% | 0,40% | 0,40% | 0,40% | 0,40% |
| | beta carotene (provitamine A) | 0,003% | 0,003% | 0,003% | 0,003% | 0,003% |
| huiles | huile coco | 6% | 6% | 6% | 6% | 6% |
| | huile olive | 3% | 3% | 3% | 3% | 3% |
| | huile sésame | 2% | 2% | 2% | 2% | 2% |
| | huile bourrache | 0,50% | 0,50% | 0,50% | 0,50% | 0,50% |
| | huile onagre | 0,50% | 0,50% | 0,50% | 0,50% | 0,50% |
| parfum | | ND | ND | ND | ND | ND |

| | | | | | | |
|---|---|---|---|---|---|---|
| Compositions des huiles solaires, les proportions étant en poids par rapport au poids total de l'huile | | | | | | |

## Revendications

1. Composition cosmétique ou dermatologique comprenant :
- à titre de filtres solaires : de l'ethylhexyl triazone, du diethylamino hydroxybenzoyl hexyl benzoate et du bis-ethylhexyloxyphenol methoxyphenyl triazine,
- à titre de vitamines ou précurseur de vitamines :
du tocopherol, un ester de celui-ci ou un mélange de ceux-ci,
de la vitamine F,
un éther de vitamine C, un ester de vitamine C, un ester phosphorique de vitamine C ou un mélange de ceux-ci, et
du beta-carotene, et
- à titre d'huiles : de l'huile d'olive, de l'huile de coco et de l'huile de bourrache.

2. Composition selon la revendication 1, comprenant :
- de 0,5% à 8,0% en poids, de préférence de 1,0 à 6,0% en poids, d'ethylhexyl triazone,
- du diethylamino hydroxybenzoyl hexyl benzoate, dans une proportion telle que le ratio massique du diethylamino hydroxybenzoyl hexyl benzoate sur l'ethylhexyl triazone est de 0,3 à 1,5, de préférence de 0,4 à 1,3, et
- du bis-ethylhexyloxyphenol methoxyphenyl triazine, dans une proportion telle que le ratio massique du bis-ethylhexyloxyphenol methoxyphenyl triazine sur l'ethylhexyl triazone est de 0,1 à 1,5, de préférence de 0,2 à 1,1.

3. Composition selon la revendication 1 ou 2, comprenant :
- de 0,9 à 2,0% en poids, de préférence de 1,0 à 1,6% en poids, de tocopherol, et/ou
- de 0,1 à 2,0% en poids, de préférence de 0,2 à 1,5% en poids, d'ester de tocopherol, et/ou
- de 0,2 à 1,5 % en poids, de préférence de 0,5 à 1,3% en poids, de vitamine F, et/ou
- de 0,1 à 1,0% en poids, de préférence de 0,2 à 0,4% en poids, d'éther de vitamine C, d'ester de vitamine C, d'ester phosphorique de vitamine C ou de mélange de ceux-ci, et/ou
- de 0,0001 à 0,01 en poids, de préférence de 0,0005 à 0,005% en poids, de beta-carotene.

4. Composition selon l'une quelconque des revendications 1 à 3, comprenant :
- 0,5 à 5,0% en poids, de préférence de 1,0 à 4,0% en poids, d'huile d'olive, et/ou
- 0,5 à 10,0% en poids, de préférence de 1,0 à 7,0% en poids, d'huile de coco, et/ou
- 0,1 à 2,0% en poids, de préférence de 0,2 à 1,0% en poids, d'huile de bourrache.

5. Composition selon l'une quelconque des revendications 1 à 4, comprenant en outre, à titre d'huiles :
- de l'huile de sésame, de préférence dans une proportion de 0,5 à 5% en poids, de préférence de 1,0 à 3,0% en poids, et/ou
- de l'huile d'onagre, de préférence dans une proportion de 0,1 à 2,0% en poids, de préférence de 0,2 à 1,0% en poids.

6. Composition selon l'une quelconque des revendications 1 à 5, comprenant en outre, à titre de filtres solaires :
- de l'ethylhexylsalicylate, de préférence dans une proportion telle que le ratio massique de l'ethylhexylsalicylate sur l'ethylhexyl triazone est de 0,2 à 2,0, de préférence de 0,5 à 1,5, et
- de l'ethylhexyl dimethyl PABA, de préférence dans une proportion telle que le ratio massique de l'ethylhexyl dimethyl PABA sur l'ethylhexyl triazone est de 0,1 à 1,5, de préférence de 0,3 à 1,3.

7. Composition selon l'une quelconque des revendications 1 à 6, comprenant en outre, à titre de vitamine, du panthenol, notamment de 0,1 à 2,0% en poids, de préférence de 0,4 à 1,0% en poids, de panthenol.

8. Composition selon l'une quelconque des revendications 1 à 7, comprenant en outre un extrait d'aloe vera.

9. Composition selon l'une quelconque des revendications 1 à 8, exempte :
- d'oxyde de zinc, et/ou
- de dioxyde de titane, et/ou
- de nanoparticules de taille moyenne de 1 à 100 nm, telle que mesurée par microscopie au balayage électronique, et/ou
- d'acide ethylenediaminetetraacétique (EDTA), et/ou
- d'hydroxytoluene butylé (BHT), et/ou
- de polysiloxanes, et/ou
- de propylene glycol, et/ou
- de dérivés de vitamine A choisis parmi l'acide rétinoïque, le rétinol, le rétinal et le rétinaldéhyde, et/ou
- d'huile minérale.

10. Composition selon l'une quelconque des revendications 1 à 9, se présentant sous forme d'un gel aqueux ou huileux, d'une émulsion de type E/H ou H/E, ou d'un liquide monophasique, de préférence sous forme d'une émulsion de type H/E ou d'une huile.
